(19) Europäisches Patentamt
European Patent Office
Office européen des brevets

(11) Veröffentlichungsnummer: **0 009 205**
**B2**

# (12) NEUE EUROPÄISCHE PATENTSCHRIFT

(45) Veröffentlichungstag der neuen Patentschrift: **24.07.85**

(51) Int. Cl.⁴: **C 07 C 63/10**, C 07 C 51/58

(21) Anmeldenummer: **79103410.1**

(22) Anmeldetag: **12.09.79**

(54) **Verfahren zur Herstellung von gegebenenfalls substituiertem Benzoylchlorid.**

(30) Priorität: **23.09.78 DE 2841541**

(43) Veröffentlichungstag der Anmeldung:
**02.04.80 Patentblatt 80/7**

(45) Bekanntmachung des Hinweises auf die Patenterteilung:
**30.12.81 Patentblatt 81/52**

(45) Bekanntmachung des Hinweises auf die Entscheidung über den Einspruch:
**24.07.85 Patentblatt 85/30**

(84) Benannte Vertragsstaaten:
**DE FR GB IT NL**

(56) Entgegenhaltungen:
**keine**

(73) Patentinhaber: **BAYER AG, Konzernverwaltung RP Patentabteilung, D-5090 Leverkusen 1 Bayerwerk (DE)**

(72) Erfinder: **Böckmann, Walter, Dr., Fabritiusstrasse 19, D-4150 Krefeld 11 (DE)**
Erfinder: **Lipper, Karl-August, Dr., Deswatinesstrasse 14, D-4150 Krefeld 1 (DE)**
Erfinder: **Brühne, Friedrich, Dr., Fabritiusstrasse 24, D-4150 Krefeld 11 (DE)**

EP 0 009 205 B2

**Beschreibung**

Die Erfindung betrifft ein Verfahren zur Herstellung von gegebenenfalls substituiertem Benzoylchlorid durch Umsetzung von gegebenenfalls substituiertem Benzotrichlorid oder einem Gemisch aus gegebenenfalls substituiertem Benzotrichlorid und gegebenenfalls substituiertem Benzoylchlorid mit gegebenenfalls substituierter Benzoesäure und/oder Wasser in Gegenwart von Katalysatoren.

Es ist bekannt, Benzoylchlorid durch Umsetzung von äquimolaren Mengen Benzotrichlorid und Benzoesäure in Gegenwart von Zinkchlorid herzustellen (DE-PS 11 494).

Weiterhin ist bekannt, Benzoylchlorid durch Umsetzung von äquimolaren Mengen Benzotrichlorid mit Wasser in Gegenwart von Eisenchlorid darzustellen (DE-PS 331 696).

Ein weiteres Verfahren zur Herstellung von Benzoylchlorid ist aus der US-PS 1 557 154 bekannt. Danach wird Benzotrichlorid mit Wasser bei erhöhter Temperatur in Gegenwart eines suspendierten Katalysators umgesetzt, wobei die Menge des zugegebenen Wassers nicht mehr als 110% der für die Umsetzung theoretisch benötigten Wassermenge betragen soll. Würde mehr Wasser bei der Umsetzung eingesetzt, so würde sich zwar die Produktqualität verbessern, aber gleichzeitig auch die Ausbeute an Benzoylchlorid verringern (vgl. Seite 2, Zeilen 14 bis 21). Nach dem Verfahren der US-PS werden Ausbeuten von 70 bis 85% erhalten (vgl. Seite 2, Zeilen 27 und 85, 86).

Gemäss der französischen Offenlegungsschrift 20 66 768 werden Carbonsäurealkylester der Benzolreihe mit Trichlormethylbenzolen zu den entsprechenden Carbonsäurechloriden in Gegenwart von Lewis- oder Brönsted-Säuren umgesetzt.

Die Trichlormethylbenzole werden dabei in einem Überschuss eingesetzt (vgl. Seite 3, Zeilen 37 bis 40 und Seite 4, Zeilen 1 bis 4). Bei diesem Verfahren werden Ausbeuten an Benzoylchlorid von 70 bis 95% erreicht.

Es wurde nun ein Verfahren zur Herstellung von gegebenenfalls substituiertem Benzoylchlorid aus gegebenenfalls substituiertem Benzotrichlorid bei erhöhter Temperatur in Gegenwart von sauren Katalysatoren und mit einem Überschuss an gegebenenfalls substituierter Benzoesäure und/oder Wasser, gefunden, das dadurch gekennzeichnet ist, dass man den Katalysator in Form einer wässrigen Lösung einsetzt, wobei man das gegebenenfalls substituierte Benzotrichlorid oder ein Gemisch aus dem gegebenenfalls substituierten Benzotrichlorid und dem gegebenenfalls substituierten Benzoylchlorid mit einem Überschuss von 1 bis 20 Mol-% (bezogen auf eingesetztes, gegebenenfalls substituiertes Benzotrichlorid) an gegebenenfalls substituierter Benzoesäure und/oder Wasser umsetzt, die bei der Umsetzung entstehenden chlorwasserstoffhaltigen Abgase mit dem eingesetzten, gegebenenfalls substituierten Benzotrichlorid oder dem eingesetzten Gemisch aus gegebenenfalls substituiertem Benzotrichlorid und gegebenenfalls substituiertem Benzoylchlorid gewaschen werden und man das nach der Destillation des gegebenenfalls substituierten Benzoylchlorids verbleibende Sumpfprodukt mit einem 3 bis 150%igen molaren Überschuss an gegebenenfalls substituiertem Benzotrichlorid, bezogen auf die im Sumpfprodukt vorhandene Menge an gegebenenfalls substituierter Benzoesäure, umsetzt und das nach der Destillation daraus erhaltene Gemisch aus gegebenenfalls substituiertem Benzotrichlorid und gegebenenfalls substituiertem Benzoylchlorid als Einsatzprodukt in die Reaktion zurückführt.

Als gegebenenfalls substituiertes Benzotrichlorid kann ein der allgemeinen Formel

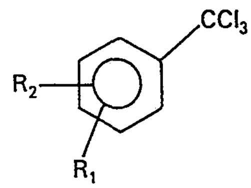

worin $R_1$ und $R_2$ gleich oder verschieden sein können und für Wasserstoff, Halogen, Nitro- oder Alkylgruppen stehen,
entsprechendes Benzotrichlorid in das erfindungsgemässe Verfahren eingesetzt werden.

Als gegebenenfalls substituierte Benzoesäure kann eine der allgemeinen Formel

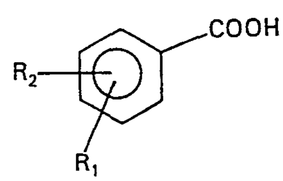

worin $R_1$ und $R_2$ die oben angegebene Bedeutung haben, entsprechende Benzoesäure eingesetzt werden.

Als Reste $R_1$ und $R_2$ kommen beispielsweise Kohlenwasserstoffe mit bis zu 6 C-Atomen in Betracht, z.B. Alkylreste, wie Methyl, Äthyl, n-Propyl, Isopropyl, n-Butil, Isobutyl, tert.-Butyl, n-Pentyl, Isopentyl, 2-Methylpentyl, 3-Methylpentyl, n-Hexyl, Isohexil sowie Cyclohexyl.

Als Halogene seien Fluor, Chlor, Brom, bevorzugt Chlor genannt.

Bevorzugt werden nach dem erfindungsgemässen Verfahren Benzotrichlorid, o-Chlor- oder p-Chlor-benzotrichlorid umgesetzt.

Das erfindungsgemässe Verfahren wird in Gegenwart von sauren Katalysatoren in Form einer wässrigen Lösung durchgeführt.

Zum Beispiel seien folgende saure Katalysatoren genannt:
$HClO_4$, $H_2SO_4$, $H_2S_2O_7$, $H_3PO_4$, $H_4P_2O_7$, aromatische oder aliphatische Sulfonsäuren mit bis zu 15 C-Atomen, wie p-Toluolsulfonsäure, Nonafluor-butansulfonsäure, Naphthalinsulfonsäure sowie Halogenide der Elemente Bor, Aluminium, Gallium, Germanium, Zinn, Arsen, Antimom, Wismut, Kupfer, Zink, Cadmium, Titan, Zirkon,

Vanadium, Niob, Chrom, Molybdän, Wolfram, Eisen, Cobalt, Nickel.

Bevorzugt werden $H_2SO_4$, $H_3PO_4$, $FeCl_2$, $ZnCl_2$ und/oder $FeCl_3$ in das erfindungsgemässe Verfahren eingesetzt.

Nach dem erfindungsgemässen Verfahren ist es möglich, die verwendeten Metallchloride nicht als solche direkt in das Verfahren einzusetzen, sondern die entsprechenden Oxide, Hydroxide und/oder Carbonate, die dann mit dem während der Umsetzung entstehenden Chlorwasserstoff die entsprechenden Metallchloride bilden.

Neben den Oxiden, Hydroxiden und/oder Carbonaten können auch Metallsalze anderer Säuren, wie der Schwefelsäure, Phosphorsäure, Essigsäure, Ölsäure sowie der Naphthensäure oder die Metalle selbst, in das erfingungsgemässe Verfahren eingesetzt werden.

Im allgemeinen verwendet man die Katalysatoren, die einzeln oder im Gemisch untereinander eingesetzt werden können, in einer Menge von etwa 0,005 bis 10 Gew.-%, vorzugsweise in einer Menge von 0,02 bis 3 Gew.-%, bezogen auf gebildetes Benzoyltrichlorid.

Die Menge des Katalysators in der wässrigen Lösung beträgt etwa 0,2 bis 98 Gew.-%, bevorzugt 0,5 bis 30 Gew.-%.

Nach dem erfindungsgemässen Verfahren wird das gegebenenfalls substituierte Benzotrichlorid oder das Gemisch aus dem gegebenenfalls substituierten Benzotrichlorid und dem gegebenenfalls substituierten Benzoylchlorid mit einem Überschuss von etwa 1 bis 20 Mol-% (bezogen auf eingesetztes, gegebenenfalls substituiertes Benzotrichlorid) bevorzugt mit einem Überschuss von 3 bis 8 Mol-%, an gegebenenfalls substituierter Benzoesäure und/oder Wasser umgesetzt.

Wird die Umsetzung gleichzeitig mit gegebenenfalls substituierter Benzoesäure und Wasser durchgeführt, kann das Gewichtsverhältnis von gegebenenfalls substituierter Benzoesäure zu Wasser jeden denkbaren Wert einnehmen, ohne dass dadurch die Reaktion negativ beeinflusst würde.

Die Zusammensetzung des in das erfindungsgemässe Verfahren eingesetzten Gemisches aus gegebenenfalls substituiertem Benzotrichlorid und gegebenenfalls substituiertem Benzoylchlorid kann in weiten Grenzen variieren. Vorzugsweise werden Gemische aus etwa 5 bis 99%, bevorzugt 20 bis 99%, gegebenenfalls substituiertem Benzotrichlorid und etwa 1 bis 95%, bevorzugt 1 bis 80%, gegebenenfalls substituiertem Benzoylchlorid eingesetzt.

Die Umsetzung wird üblicherweise bei Temperaturen im Bereich von etwa 70 bis 200°C, bevorzugt 120 bis 160°C, durchgeführt.

Die Reaktion kann bei Normaldruck, bei vermindertem Druck oder bei erhöhtem Druck durchgeführt werden, vorzugsweise erfolgt die Reaktion bei Normaldruck.

Die bei der Umsetzung entstehenden clorwasserstoffhaltigen Abgase werden erfindungsgemäss mit dem einzusetzenden, gegebenenfalls substituierten Benzotrichlorid oder dem Gemisch aus gegebenenfalls substituiertem Benzotrichlorid und gegebenenfalls substituiertem Benzoylchlorid gewaschen.

Zusätzlich können die chlorwasserstoffhaltigen Abgase noch mit umgepumptem Reaktionsprodukt und/oder mit inerten, gegebenenfalls durch Halogene, wie Fluor, Chlor, Brom, bevorzugt durch Chlor, substituierten aliphatischen, cycloaliphatischen und/oder aromatischen Kohlenwasserstoffen mit bis zu 15-C-Atomen, bevorzugt bis 10 C-Atomen, gewaschen werden.

Als gegebenenfalls substituierte aliphatische Kohlenwasserstoffe seien z.B. genannt:
Tetrachlorkohlestoff, Trichloräthylen und Hexan;
als gegebenenfalls substituierte cycloaliphatische Kohlenwasserstoffe:
Cyclohexan, Methylcyclohexan und Dekalin
und als gegebenenfalls substituierte aromatische Kohlenwasserstoffe: Benzol, Chlorbenzol, Toluol, Xylol und Chlortoluol.

Bevorzugt werden Toluol, Xylol und Chlortoluol zur Wäsche der HCL-haltigen Abgase eingesetzt.

Nach einer bevorzugten Ausführungsform des erfindungsgemässen Verfahrens wird die Wäsche der chlorwasserstoffhaltigen Abgase wie folgt durchgeführt:

Die aus dem/den Reaktor(en) entweichenden HCl-haltigen Abgase werden in einem Waschturm im Gegenstrom mit der flüssigen, heissen Reaktionsmischung aus dem ersten Reaktor gewaschen.

Das Abgas wird dann einem zweiten Waschturm zugeführt, wo es bei Raumtemperatur mit dem gegebenenfalls substituierten Benzotrichlorid oder dem Gemisch aus gegebenenfalls substituiertem Benzotrichlorid und gegebenenfalls substituiertem Benzoylchlorid im Gegenstrom gewaschen wird.

Anschliessend gelangt das Abgas in einen dritten Waschturm, wo es bei tiefen Temperaturen inerten, gegebenenfalls substituierten aromatischen und/oder aliphatischen Lösungsmitteln gewaschen wird. Bevorzugt wird als inertes Lösungsmittel Toluol eingesetzt.

Durch die erfindungsgemässe Wäsche erhält man ein chlorwasserstoffhaltiges Abgas, das praktisch frei von organischen Bestandteilen wie gegebenenfalls substituiertem Benzoylchlorid, gegebenenfalls substituiertem Benzotrichlorid und gegebenenfalls substituierter Benzoesäure ist und das z.B. für eine Chlorwasserstoff-Elektrolyse geeignet ist oder durch adiabatische Absorption zu sehr reiner wässriger Salzsäure kondensiert werden kann.

Daneben werden durch die Wäsche des chlorwasserstoffhaltigen Abgases Verluste von Reaktionskomponenten, die noch im Abgas vorhanden sind, vermieden.

Im erfindungsgemässen Verfahren wird das nach der Destillation des Reaktionsproduktes verbleibende Sumpfprodukt, das sich aus etwa 20 bis 70 Gew.-% gegebenenfalls substituiertem Benzoylchlorid, 20 bis 70 Gew.-% gegebenenfalls substituierter Benzoesäure und 0,05 bis 10

Gew.-% Katalysator zusammensetzt, mit einem molaren Überschuss an gegebenenfalls substituiertem Benzotrichlorid umgesetzt. Der molare Überschuss an gegebenenfalls substituiertem Benzotrichlorid beträgt im allgemeinen 3 bis 150%, bevorzugt 10 bis 100%, bezogen auf die im Sumpfprodukt vorhandene Menge gegebenenfalls substituierter Benzoesäure.

Die Umsetzung des Sumpfproduktes mit dem gegebenenfalls substituierten Benzotrichlorid wird im allgemeinen bei Temperaturen im Bereich von etwa 70 bis 200°C, vorzugsweise bei 120 bis 160°C, durchgeführt.

Die bei dieser Umsetzung entstehenden chlorwasserstoffhaltigen Abgase können, wie zuvor beschrieben, mit dem einzusetzenden gegebenenfalls substituierten Benzoylchlorid oder mit dem Gemisch aus gegebenenfalls substituiertem Benzoylchlorid und gegebenenfalls substituiertem Benzotrichlorid gewaschen werden. Zusätzlich kann chlorwasserstoffhaltiges Abgas mit umgepumptem Reaktionsprodukt und/oder mit den zuvor erwähnten inerten, gegebenenfalls substituierten aliphatischen, cycloaliphatischen und/oder aromatischen Kohlenwasserstoffen gewaschen werden.

Bei der Umsetzung des Sumpfproduktes mit gegebenenfalls substituiertem Benzotrichlorid ist die Zugabe eines Katalysators im allgemeinen nicht mehr erforderlich, da im Sumpfprodukt noch Katalysator vorhanden ist. Gegebenenfalls können jedoch die zuvor beschriebenen Katalysatoren zugesetzt werden, wobei die Katalysatormenge etwa 0,005 bis 10 Gew.-%, bevorzugt 0,02 bis 3 Gew.-%, bezogen auf gebildetes Benzoylchlorid, beträgt.

Nach der Reaktion des Sumpfproduktes mit dem gegebenenfalls substituierten Benzotrichlorid wird das erhaltene Reaktionsgemisch, destilliert, wobei ein Gemisch aus gegebenenfalls substituiertem Benzotrichlorid und gegebenenfalls substituiertem Benzoylchlorid erhalten wird, das als Einsatzprodukt in die Hauptreaktion zurückgeführt wird.

Das erfindungsgemässe Verfahren kann sowohl diskontinuierlich als auch kontinuierlich durchgeführt werden.

Bei der kontinuierlichen Arbeitsweise wird die Umsetzung des gegebenenfalls substituierten Benzotrichlorids oder des Gemisches aus gegebenenfalls substituiertem Benzotrichlorid und gegebenenfalls substituiertem Benzoylchlorid mit gegebenenfalls substituierter Benzoesäure und/oder Wasser vorzugsweise in mehreren miteinander verbundenen Reaktoren, besonders bevorzugt in 2 bis 5 Reaktoren, durchgeführt.

Dabei wird z.B. zur Herstellung von Benzoylchlorid oder das Gemisch aus Benzotrichlorid und Benzoylchlorid zusammen mit Benzoesäure und/oder Wasser und dem Katalysator kontinuierlich in den ersten Reaktor eindosiert. Das Reaktionsgemisch fliesst dann kontinuierlich in die anderen Reaktionsbehälter (falls mehrere vorhanden sind), wo die Umsetzung vervollständigt wird, und gelangt schliesslich in eine Destillationsanlage. Dort wird das Reaktionsgemisch kontinuierlich, vorzugsweise im Vakuum, bei etwa 12 bis 190 mbar, und Temperaturen im Bereich von etwa 70 bis 140°C destilliert, wobei ein reines, Benzotrichlorid-freies, Benzoylchlorid erhalten wird. Das Sumpfprodukt der Destillation wird bei etwa 70 bis 200°C, vorzugsweise bei 120 bis 160°C, mit einem etwa 3 bis 150%igen molaren Überschuss an Benzotrichlorid, bezogen auf die im Sumpfprodukt vorhandene Menge Benzoesäure, umgesetzt und das nach der Destillation daraus erhaltene Gemisch aus Benzotrichlorid und Benzoylchlorid als Einsatzprodukt in die Hauptreaktion zurückgegeben, wo es mit frischem Benzotrichlorid für eine weitere Umsetzung versetzt wird.

Die Umsetzung des Sumpfproduktes kann sowohl diskontinuierlich als auch kontinuierlich erfolgen. Bei der kontinuierlichen Arbeitsweise erfolgt die Reaktion üblicherweise in mehreren, hintereinandergeschalteten Reaktoren, vorzugsweise in 2 bis 5 Reaktoren.

Das bei der Umsetzung gebildete chlorwasserstoffhaltige Abgas wird, wie zuvor beschrieben mit dem gegebenenfalls substituierten Benzotrichlorid oder einem Gemisch aus gegebenenfalls substituiertem Benzotrichlorid und gegebenenfalls substituiertem Benzoylchlorid, gegebenenfalls zusätzlich mit umgepumptem Reaktionsprodukt und/oder inerten organischen Lösungsmitteln gewaschen.

Nach dem erfindungsgemässen Verfahren, das aus einer Kombination von verschiedenen Verfahrensschritten besteht, erhält man das gegebenenfalls substituierte Benzoylchlorid in einer Ausbeute von 96 bis 98% der Theorie und in einer Reinheit von über 99,5%.

Das erfindunsgemässe Verfahren weist gegenüber dem Stand der Technik folgende Vorteile auf:

Durch Verwendung eines Überschusses an gegebenenfalls substituierter Benzoesäure und/oder Wasser wird sichergestellt, dass die Umsetzung des gegebenenfalls substituierten Benzotrichlorids vollständig verläuft und dass das gebildete gegebenenfalls substituierte Benzoylchlorid stets frei von dem entsprechenden Benzotrichlorid ist. Diese Massnahme führt zur Einsparung von Destillationskosten, da die Reindestillation weniger aufwendig ist. Für viele Einsatzgebiete wird nämlich ein absolut Benzotrichlorid-freies Benzoylchlorid gefordert, das so in einfacher Weise bereitgestellt werden kann.

Weiterhin können für die Umsetzung gegebenenfalls substituierte Benzoesäure und Wasser in der gleichen Anlage ohne Veränderung des Verfahrensbedingungen gleichzeitig eingesetzt werden, ohne dass eine Begrenzung des molaren Verhältnisses von gegebenenfalls substituierter Benzoesäure zu Wasser erforderlich ist. Diese variable Verfahrensweise gestattet es, sich den Gegebenheiten der Praxis wie Verfügbarkeit von Rohstoffen, z.B. Benzoesäure, oder Absatzmöglichkeiten von Chlorwasserstoff bzw. wässriger Salzsäure kurzfristig anzupassen.

Zusätzlich wird durch die verschiedenen Massnahmen zur Wäsche des gebildeten Chlorwasserstoffes erreicht, dass mit dem Abgasstrom keine sonstigen Reaktionskomponenten verloren gehen, die Stöchiometrie der Reaktion also stets leicht einzuhalten ist, und dass das erzeugte HCl-Gas nach der Wäsche praktisch frei von organischen Verunreinigungen ist und unmittelbar zur Salzsäureelektrolyse eingesetzt oder durch Absorption in Wasser in sehr reine, wässrige Salzsäure übergeführt werden kann, die für praktisch alle Einsatzgebiete geeignet ist.

Weiterhin bewirkt die Wäsche, dass die Abgasleitungen nicht mehr durch Feststoffe verstopft werden und so den Betriebsablauf stören.

Zudem ist das erfindungsgemässe Verfahren besonders umweltfreundlich, da es keine verunreinigenden Abwässer oder Abgase erzeugt. Der erhaltene hochreine Chlorwasserstoff kann in Chlorgas umgewandelt und damit über die Herstellung der Benzotrichloride wieder in den Prozess zurückgeführt werden. Durch die geschilderten Massnahmen wird die Wirtschaftlichkeit der Benzoylchlorid-Herstellung erheblich gesteigert.

Im Unterschied zu dem in der US-PS 1 557 154 beschriebenen Verfahren ist es bei dem erfindungsgemässen Verfahren möglich, mit einem grösseren Überschuss an Wasser zu arbeiten, ohne dass dadurch die Ausbeute oder die Produktqualität des erhaltenen Benzoylchlorids sich verschlechtern bzw. verringern würde.

Trotz des in der US-PS 1 557 154 aufgebauten Vorurteils, grössere Mengen an Wasser bei der Verseifung von Benzotrichlorid wegen der Bildung von unerwünschter Benzoesäure zu vermeiden, gelingt es nach dem erfindungsgemässen Verfahren überraschenderweise, Benzoylchlorid in den zuvor erwähnten sehr guten Ausbeuten und ausserordentlich hohen Reinheiten herzustellen.

Das nach dem erfindungsgemässen Verfahren hergestellte, gegebenenfalls substituierte Benzoylchlorid ist ein wertvolles Zwischenprodukt für die Herstellung von gegebenenfalls substituiertem Benzoylperoxid, Benzophenon, Benzylbenzoat, Benzoylcellulose, Benzamid sowie von Farbstoffen und Arzneimitteln (vgl. Ullmanns Enzyklopädie der technischen Chemie, 4. Auflage, Band 8, Seite 372-373, Verlag Chemie, Weinheim 1974; Kirk-Othmer, Encyclopedia of Chemical Technology, 2. Auflage, Band 3, Seite 430-432, Interscience Publishers, New York, London, Sydney 1964).

Beispiel 1

4887 g (25,0 Mol) Benzotrichlorid werden in einem 5-l-Kolben mit Rührer, Rückflusskühler und Tropftrichter auf 150°C erhitzt. Hierauf lässt man bei dieser Temperatur innerhalb von 5 Stunden 483 g 3%ige wässrige Schwefelsäure zufliessen. Nach Beendigung der Schwefelsäurezugabe wird das Reaktionsprodukt noch 2 Stunden bei 150°C gerührt. Aufgrund der Siedeanalyse beträgt der Gehalt an Benzeosäure und Benzoesäureanhydrid im rohen Benzoylchlorid 3,8 Gew.-%. Die Destillation des Umsetzungsprodukts im Vakuum bei 50 mbar ergibt 3255 g (92,6% der Theorie) reines, benzotrichloridfreies Benzoylchlorid und 210 g Destillationssumpf. Dieser wird mit 260 g (1,3 Mol) Benzotrichlorid in einem Rührgefäss mit Rückflusskühler auf 150°C erhitzt und 2 Stunden bei dieser Temperatur gehalten. Die Destillation bei 50 mbar liefert 368 g eines Gemisches aus Benzotrichlorid und Benzoylchlorid mit einem Benzotrichlorid-Gehalt von 13,0 Gew.-%. Dieses Produkt kann bei dem nächsten Ansatz als Ausgangsstoff wieder eingesetzt werden. Unter Berücksichtigung des zurückgewonnenen Gemisches aus Benzotrichlorid und Benzoylchlorid erhöht sich die Ausbeute an reinem Benzoylchlorid auf 97,3% der Theorie.

Das bei der Hauptreaktion und der Destillationssumpfaufarbeitung entweichende HCl-Abgas wird in einem mit Raschigringen gefüllten Waschturm mit 4887 g Benzotrichlorid, die mittels einer Pumpe im Kreis geführt werden, gewaschen und anschliessend zu einer ca. 25%igen wässrigen Salzsäure kondensiert. Die Salzsäure weist einen Gehalt an organisch gebundenem Kohlenstoff von 22 ppm auf. Das für die Abgaswäsche verwendete Benzotrichlorid wird wie das zurückgewonnene Gemisch aus Benzotrichlorid und Benzoylchlorid für den nächsten Ansatz als Ausgangsstoff wieder eingesetzt.

Beispiel 2

In das erste Gefäss einer Kaskade aus vier Reaktionsgefässen, von denen jedes ein Nutzvolumen von 2,5 l besitzt, werden mittels Dosierpumpe 830 g/h Benzotrichlorid, 345 g/h eines Gemisches aus Benzotrichlorid und Benzoylchlorid (Benzotrichlorid 22,6 Gew.-%), 403 g/h flüssige Benzoesäure und 37 g/h einer 2,5%igen Lösung von Eisen(II)chlorid in Wasser eingeführt. Das Gemisch aus Benzotrichlorid und Benzoylchlorid fällt bei der Aufarbeitung des Sumpfes der Benzoylchlorid-Destillation an, wie weiter unten noch beschrieben wird. Das Reaktionsprodukt des ersten Gefässes läuft über einen seitlichen Abfluss frei in das zweite Gefäss, von dort in das dritte und weiter in das vierte Gefäss ab. Der Inhalt der vier Reaktionsgefässe wird gerührt und bei einer Temperatur von 150°C gehalten.

Das aus dem vierten Reaktionsgefäss ablaufende Produkt gelangt über einen Kühler in ein Zwischengefäss. Nach 24 Stunden Laufzeit hat sich in den vier Reaktionsgefässen ein stationärer Gleichgewichtszustand eingestellt. Die gaschromatographische Analyse des aus dem vierten Gefäss ablaufenden rohen Benzoylchlorids zeigt, dass kein Benzotrichlorid mehr vorhanden ist. Aufgrund der Siedeanalyse enthält das Rohprodukt 6,0 Gew.-% Benzoesäure und Benzoesäureanhydrid. Durch kontinuierliche Destillation des Rohproduktes bei 50 mbar werden pro Stunde 1198 g reines, benzotrichloridfreies Benzoylchlorid und 172 g Destillationssumpf erhalten.

Die in 12 Stunden angefallene Menge an Destillationssumpf (2064 g) wird mit 2718 g Benzotrichlorid in einem Rührgefäss mit Rückflusskühler auf 150°C erhitzt und 2 Stunden bei dieser Temperatur gehalten. Die Destillation bei 50 mbar liefert 4140 g eines Gemisches aus Benzotrichlorid und Benzoylchlorid mit einem Benzotrichloridgehalt von 22,6 Gew.-%, das in das erste Gefäss der vierstufigen Kaskade wieder eindosiert wird.

Das aus den vier Reaktionsgefässen der Kaskade und aus dem Reaktionsgefäss für die Umsetzung des Sumpfes der Benzoylchlorid-Destillation mit Benzotrichlorid entweichende HCl-Abgas wird vereinigt und in einem Waschturm zunächst mit dem umgepumpten Reaktionsprodukt des ersten Reaktionsgefässes und dann in einem zweiten Waschturm mit dem für die Hauptreaktion eingesetzten Benzotrichlorid und dem Gemisch aus Benzotrichlorid und Benzoylchlorid gewaschen. Nach einer weiteren Wäsche in einem dritten Waschturm mit Toluol, das eine Temperatur von −25°C besitzt, wird das HCl-Abgas zu ca. 30%iger Salzsäure kondensiert. Diese Salzsäure weist einen Gehalt an organisch gebundenem Kohlenstoff von max. 5 ppm auf.

Die Gesamtausbeute an Benzoylchlorid, bezogen auf eingesetzte Benzoesäure und Benzotrichlorid, beträgt 97,9% der Theorie.

Beispiel 3

4599 g (20,0 Mol) p-Chlor-benzotrichlorid werden in einem 5-l-Kolben mit Rührer, Rückflusskühler und Tropftrichter auf 150°C erhitzt. Dann lässt man bei dieser Temperatur innerhalb von 4 Stunden eine Lösung von 2,4 g Eisen(II)-sulfat in 378 g Wasser zufliessen. Anschliessend wird das Reaktionsprodukt noch 2 Stunden bei 150°C gerührt. Die Destillation im Vakuum bei 70 mbar ergibt 3213 g (91,8% der Theorie) reines, p-Chlorbenzotrichloridfreies p-Chlor-benzoylchlorid und 226 g Destillationssumpf. Dieser wird mit 286 g (1,24 Mol) p-Chlor-benzotrichlorid in einem Rührgefäss mit Rückflusskühler auf 150°C erhitzt und 2 Stunden bei dieser Temperatur gehalten. Die Destillation im Vakuum bei 70 mbar liefert 410 g eines Gemisches aus p-Chlor-benzotrichlorid und p-Chlor-benzoylchlorid mit einem p-Chlorbenzotrichloridgehalt von 8,8 Gew.-%. Dieses Produkt kann bei dem nächsten Ansatz als Ausgangsstoff wieder eingesetzt werden. Unter Berücksichtigung des zurückgewonnenen Gemisches aus p-Chlor-benzotrichlorid und p-Chlorbenzoylchlorid erhöht sich die Ausbeute an reinem p-Chlor-benzoylchlorid auf 96,9% der Theorie.

Das bei der Hauptreaktion und der Destillationsaufarbeitung entweichende HCl-Gas wird in einem mit Raschigringen gefüllten Waschturm mit 4599 g p-Chlor-benzotrichlorid, die mittels einer Pumpe im Kreis geführt werden, gewaschen und anschliessend zu einer ca. 26%igen wässrigen Salzsäure kondensiert. Die Salzsäure weist einen Gehalt an organisch gebundenem Kohlenstoff von 18 ppm auf. Das für die Abgaswäsche verwendete p-Chlor-benzotrichlorid wird wie das zurückgewonnene Gemisch aus p-Chlorbenzotrichlorid und p-Chlor-benzoylchlorid für den nächsten Ansatz als Ausgangsstoff wieder eingesetzt.

**Patentansprüche**

1. Verfahren zur Herstellung von gegebenenfalls substituiertem Benzoylchlorid aus gegebenenfalls substituiertem Benzotrichlorid bei erhöhter Temperatur in Gegenwart von sauren Katalysatoren und mit einem Übeschuss an gegebenenfalls substituierter Benzoesäure und/oder Wasser, dadurch gekennzeichnet, dass man den Katalysator in Form eine wässrigen Lösung einsetzt, wobei man das gegebenenfalls substituierte Benzotrichlorid oder ein Gemisch aus dem gegebenenfalls substituierten Benzotrichlorid und dem gegebenenfalls substituierten Benzoylchlorid mit einem Überschuss von 1 bis 20 Mol-% (bezogen auf eingesetztes, gegebenenfalls substituiertes Benzotrichlorid) an gegebenenfalls substituierter Benzoesäure und/oder Wasser umsetzt, die bei der Umsetzung entstehenden chlorwasserstoffhaltigen Abgase mit dem eingesetzten, gegebenenfalls substituierten Benzotrichlorid oder dem eingesetzten Gemisch aus gegebenenfalls substituiertem Benzotrichlorid und gegebenenfalls substituiertem Benzoylchlorid gewaschen werden und man das nach der Destillation des gegebenenfalls substituierten Benzoylchlorids verbleibende Sumpfprodukt mit einem 3 bis 150%igen molaren Überschuss an gegebenenfalls substituiertem Benzotrichlorid, bezogen auf die im Sumpfprodukt vorhandene Menge an gegebenenfalls substituierter Benzoesäure, umsetzt und das nach der Destillation daraus erhaltene Gemisch aus gegebenenfalls substituiertem Benzotrichlorid und gegebenenfalls substituiertem Benzoylchlorid als Einsatzprodukt in die Reaktion zurückführt.

2. Verfahren nach Anspruch 1, dadurch gekennzeichnet, dass man als gegebenenfalls substituiertes Benzotrichlorid ein der allgemeinen Formel:

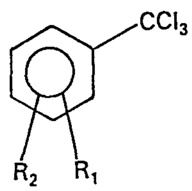

worin

$R_1$ und $R_2$ gleich oder verschieden sein können und für Wasserstoff, Halogen, Nitro- oder Alkylgruppen stehen,

entsprechendes Benzotrichlorid einsetzt.

3. Verfahren nach Ansprüchen 1 oder 2, dadurch gekennzeichnet, dass man Benzotrichlorid, o-Chlor- oder p-Chlor-benzotrichlorid einsetzt.

4. Verfahren nach Ansprüchen 1 bis 3, dadurch gekennzeichnet, dass man als gegebenenfalls substituierte Benzoesäure eine der allgemeinen Formel:

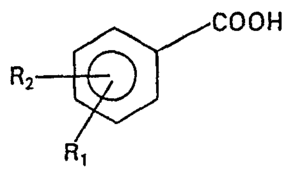

worin
$R_1$ und $R_2$ gleich oder verschieden sein können und für Wasserstoff, Halogen, Nitro- oder Alkylgruppen stehen,
entsprechende Benzoesäuren einsetzt.

5. Verfahren nach Ansprüchen 1 bis 4, dadurch gekennzeichnet, dass man das bei der Reaktion entstehende chlorwasserstoffhaltige Abgas zusätzlich mit dem umgepumpten Reaktionsprodukt und/oder mit inerten, gegebenenfalls substituierten aliphatischen, cycloaliphatischen und/oder aromatischen Kohlenwasserstoffen mit bis zu 15 C-Atomen wäscht.

6. Verfahren nach Anspruch 5, dadurch gekennzeichnet, dass man das chlorwasserstoffhaltige Abgas mit Toluol, Chlortoluol und/oder Xylol wäscht.

**Claims**

1. Process for the preparation of optionally substituted benzoyl chloride from optionally substituted benzotrichloride at elevated temperature in the presence of acid catalysts and with an excess of optionally substituted benzoic acid and/or water, charcterised in that the catalyst is used in the form of an aqueous solution, the optionally substituted benzotrichloride or a mixture of the optionally substituted benzotrichloride and the optionally substituted benzoyl chloride being reacted with an excess of 1 to 20 mol % (based on the optionally substituted benzotrichloride employed) of optionally substituted benzoic acid and/or water, the hydrogen-chloride-containing off-gases formed during the reaction being washed with the optionally substituted benzotrichloride employed or the mixture optionally substituted benzotrichloride and optionally substituted benzoyl chloride employed and the bottom product remaining after the distillation of the optionally substituted benzoyl chloride being reacted with a 3 to 150% molar excess of optionally substituted benzotrichloride, based on the quantity of optionally substituted benzoic acid present in the bottom product, and the mixture of optionally substituted benzotrichloride and optionally substituted benzoyl chloride obtained therefrom after the distillation being recycled as a feed product into the reaction.

2. Process according to Claim 1, characterised in that the optionally substituted benzotrichloride employed is a benzotrichloride which corresponds to the general formula

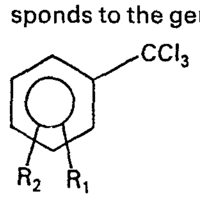

wherein
$R_1$ and $R_2$ can be identical or different and represent hydrogen, halogen or nitro or alkyl groups.

3. Process according to Claims 1 or 2, characterised in that benzotrichloride, or o-chloro- or p-chloro-benzotrichloride is employed.

4. Process according to Claims 1 to 3, characterised in that the optionally substituted benzoic acid employed is a benzoic acid which corresponds to the general formula

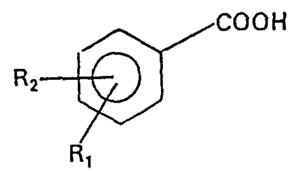

wherein
$R_1$ and $R_2$ can be identical or different and represent hydrogen, halogen or nitro or alkyl groups.

5. Process according to Claims 1 to 4, characterised in that the hydrogen-chloride-containing off-gas evolved during the reaction is additionally washed with the circulated reaction product and/or with inert, optionally substituted aliphatic, cycloaliphatic and/or aromatic hydrocarbons with up to 15 C atoms.

6. Process according to Claim 5, characterised in that the hydrogen-chloride-containing off-gas is washed with toluene, chlorotoluene and/or xylene.

**Revendications**

1. Procédé de production de chlorure de benzoyle éventuellement substitué à partir de chlorure de benzényle éventuellement substitué, à température élevée en présence de catalyseurs acides et avec un excès d'acide benzoïque éventuellement substitué et/ou d'eau, caractérisé en ce qu'on utilise le catalyseur sous la forme d'une solution aqueuse en faisant alors réagir le chlorure de benzényle éventuellement substitué ou un mélange du chlorure de benzényle éventuellement substitué et du chlorure de benzoyle éventuellement substitué avec un excès 1 à 20 moles % (par rapport au chlorure de benzényle éventuellement substitué utilisé) d'acide benzoïque éventuellement substitué et/ou d'eau, on lave les gaz résiduaires contenant du chlorure d'hydrogène formés au cours de la réaction avec le chlorure de benzényle éventuellement substitué utilisé ou avec le mélange utilisé de chlorure de benzényle éventuellement substitué et de chlorure de benzoyle éventuellement substitué et on fait réagir le produit de queue restant après la distillation du chlorure de benzoyle éventuellement substitué, avec un excès molaire de 3 à 150 % de chlorure de benzényle éventuellement substitué, par rapport à la quantité, présente dans le produit de queue, d'acide benzoïque éventuellement substitué et on recycle comme charge dans

la réaction le mélange résultant après distillation, de chlorure de benzényle éventuellement substitué et de chlorure de benzoyle éventuellement substitué.

2. Procédé suivant la revendication 1, caractérisé en ce qu'on utilise comme chlorure de benzényle éventuellement substitué un chlorure de benzényle répondant à la formule générale:

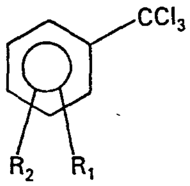

dans laquelle
$R_1$ et $R_2$ peuvent être égaux ou différents et représentent l'hydrogène, un halogène, des groupes nitro ou des groupes alkyle.

3. Procédé suivant la revendication 1 ou 2, caractérisé en ce qu'on utilise chlorure de benzényle, du chlorure de o-chlorobenzényle ou du chlorure de p-chlorobenzényle.

4. Procédé suivant les revendications 1 à 3, caractérisé en ce qu'on utilise comme acide benzoïque éventuellement substitué un acide benzoïque correspondant à la formule générale:

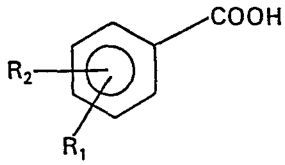

dans laquelle
$R_1$ et $R_2$ peuvent être égaux ou différents et représentent l'hydrogène, un halogène, des groups nitro ou des groupes alkyle.

5. Procédé suivant les revendications 1 à 4, caractérisé en ce qu'on lave en outre les gaz résiduaires contenant du chlorure d'hydrogène formés au cours de la réaction avec le produit réactionnel circulant par pompage et/ou avec des hydrocarbures aliphatiques, cycloaliphatiques et/ou aromatiques inertes éventuellement substitués ayant jusqu'à 15 atomes de carbone.

6. Procédé suivant la revendication 5, caractérisé en ce qu'on lave les gaz résiduaires contenant du chlorure d'hydrogène avec du toluène, du chlorotoluène et/ou du xylène.